# EUROPEAN PATENT APPLICATION

(11) **EP 3 865 502 A1**
(43) Date of publication of application: **18.08.2021**
(21) Application number: 21167932.9
(22) Date of filing: 23.12.2010
(51) Int. Cl.: C07J 41/00, A61K 31/57, A61P 5/36

(54) **COMPOSITIONS AND METHODS FOR NON-TOXIC DELIVERY OF CDB-2914**

(30) Priority: 22.03.2010 US 31626310 P
(62) Divisional of application: 10807575.5
(71) Applicant: Allergan Pharmaceuticals International Limited, Coolock, Dublin, D17 E400 (IE)
(72) Inventor: PODOLSKI, Joseph S., The Woodlands, TX, 77381 (US); WIEHLE, Ronald D., Houston, TX, 77059 (US)
(74) Representative: Danner, Stefan

(57) **Abstract**

The present invention relates to a pharmaceutical composition comprising 17.alpha.-acetoxy-11.beta.-(4-N,N-dimethylaminophenyl)-19-norpregna-4,9-diene-3,20-dione (CDB-2914) for use in treating endometriosis or pain associated therewith, or uterine fibroids, by daily administration of 5 mg to 20 mg thereof to the vaginal mucosa of a female patient in need thereof for a period of at least 4 months. 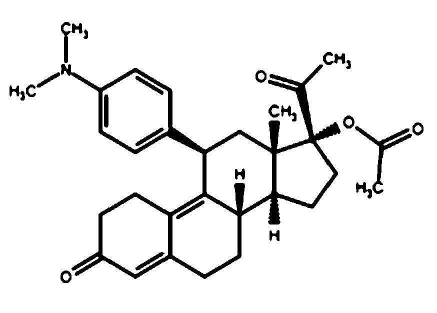 CDB-2914

## Description

This application claims the benefit, under 35 USC 119(e), of U.S. Provisional Patent Application No. 61/316,263, filed on March 22, 2010, the entire contents of which are hereby incorporated by reference.

### FIELD OF THE INVENTION

In various embodiments, the present invention relates to compositions and methods for the treatment of various hormone dependent conditions that avoid liver toxicity. In several embodiments, the present invention relates to 19-norsteroid progesterone receptor modulators with reduced liver toxicity. In another embodiment, the present invention relates to methods of administering antiprogestins to a patient in need thereof which, *inter alia,* avoid first pass metabolism of the antiprogestin.

### BACKGROUND OF THE INVENTION

The effect of the steroid hormone progesterone on the reproductive system has been well-documented. For example, progesterone is vital to establishing and maintaining pregnancy and exerts actions on various tissues of the reproductive system. The action of progesterone on tissues outside the reproductive system has been reported but is less well characterized.

Antiprogestins, compounds which inhibit the action of progesterone, have considerable potential for use in the pharmacological regulation of fertility and a variety of conditions and diseases such as breast cancer and endometriosis. The first reported antiprogestin, mifepristone (RU 486), is one of a number of 19-nortestsosterone derivatives with strong affinity for both the progesterone and glucocorticoid receptors and with antiprogestational and antiglucocorticoid activity. A variety of antiprogestins based on the 19-norprogesterone backbone have also been synthesized.

Several drawbacks are associated with the use of known antiprogestins, rendering them less than ideal for chronic administration. If these and other limitations associated with antiprogestin treatment could be improved, a significant advance in the treatment of hormone-dependent disorders would result.

### SUMMARY OF THE INVENTION

In one embodiment, the present invention provides new steroids which possess potent antiprogestational activity, minimal antiglucocorticoid activity and reduced liver toxicity. More particularly, the present invention provides compounds having the general formula: 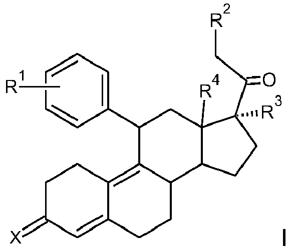 and pharmaceutically acceptable salts thereof wherein: R¹ may be at the para, ortho or meta position and is a functional group including, but not limited to, alkyl; alkenyl; cycloalkyl; cycloalkenyl; aryl; H; CH₃SO; CH₃SO₂; acyl (e.g. formyl, acetyl, propionyl, butyryl and the like); alkoxy (e.g. -OCH₃, -O(CH₂)₂CH₃, -O-CH₂-CH=CH₂); thioalkoxy; thioalkyl (e.g. - SCH₃) acyloxy (e.g. acetoxy, propanoyloxy); Si(CH₃)₃; 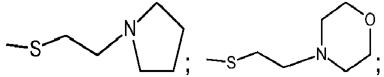 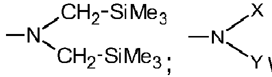 wherein X and Y are acyl; or a heterocyle preferably containing at least one nitrogen atom (e.g. aziridinyl 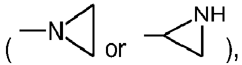 azirinyl 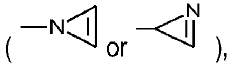 azetidinyl, pyrrolidinyl, ethoxypyrrolidinyl, methoxypyrrolidinyl, pyrrole 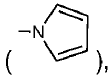 piperidinyl, ethoxypiperidinyl, pyridinyl 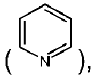 morpholinyl, ethoxymorpholinyl, oxazinyl, piperazinyl 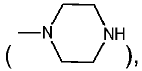 substituted piperazinyl 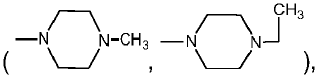 diazinyl, and an azole such as pyrazole 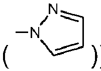 ). R² is a functional group including, but not limited to, hydrogen, halogen, alkyl, acyl, hydroxyl, akoxy (e.g., methoxy, ethoxy, vinyloxy, ethynyloxy, cyclopropyloxy, etc.), acyloxy (e.g., formyloxy, acetoxy, propionyloxy, heptanoyloxy, glycinate, etc.), alkyl carbonate, cypionyloxy, S-alkyl, S-CN, S-acyl and -OC(O)R⁶ wherein R⁶ is functional group including alkyl, alkoxyalkyl (e.g. -CH₂OCH₃) or alkoxy (-OCH₃). R³ is a functional group including but not limited to alkyl (e.g., methyl, methoxymethyl), hydroxy, alkoxy (e.g., methoxy, ethoxy, methoxymethyl, etc.), and acyloxy with the proviso that R³ is other than acetoxy or propynyl. R⁴ is a functional group including but not limited to hydrogen and alkyl. Finally, X is a functional group including but not limited to =O, =N-OR5 wherein R5 is hydrogen or alkyl, OH, CH₂, OAlk₁, and OCOAlk₂, wherein Alk₁ and Alk₂ are C1-C8 alkyl or C7-C15 aralalkyl, with the proviso that if R¹ is at the para position and is -OCH₃, -SCH₃, -NC₄H₈, -NC₅H₁₀, -NC₄H₈O, -CHO, -CH(OH)CH₃, -COCH₃, -O(CH₂)₂NC₄H₈, or -O(CH₂)₂NC₅H₁₀, X is other than =O or =N-OR5 wherein R5 is hydrogen or alkyl.

In a related embodiment, the present invention provides methods wherein compounds of general formula I (or pharmaceutical compositions comprising compounds of general formula I) are used to treat a variety of hormone (i.e., estrogen and/or progesterone) dependent conditions in a patient in need of such treatment. In a related embodiment, the compounds of general formula I are administered long term to treat a chronic hormone-dependent condition. In another related embodiment, the compounds of general formula I are administered by any route, including oral administration (i.e., administering to the gastrointestinal tract of a subject). In a preferred embodiment, the compounds of general formula I are administered to the vaginal mucosa for the long-term treatment of a chronic hormone-dependent condition.

In another embodiment, the present invention provides methods of administering compositions of one or more antiprogestins (or pharmaceutical compositions comprising one or more antiprogestins) which avoid liver toxicity. The antiprogestin may be any antiprogestin (e.g., a selective progesterone receptor modulator (SPRM), a compound of general formula I, or any other compound that inhibits the effect of progesterone), so long as the antiprogestin is administered in an amount effective to treat a progesterone-dependent condition.

In a related embodiment, a method of treating a variety of hormone dependent conditions in a patient in need of such treatment is provided comprising administration of a composition comprising one or more antiprogestins by a route that avoids first pass metabolism. Preferably the composition is administered by a route selected from the group consisting of: cutaneous, sublingual/buccal, intravascular, intramuscular, subcutaneous, inhalation, rectal, vaginal, intrauterine and topical. Most preferably, the composition is administered locally to the vaginal mucosa for the treatment of one or more hormone-dependent conditions.

Hormone-dependent conditions that may be treated by compositions of the invention include, without limitation, endometriosis and pain associated therewith, adenomyosis, endometriomas of the ovary, dysmenorrhea, endocrine hormone-dependent tumors, uterine fibroids, endometrial hyperproliferation, ovarian cancer, cervical cancer and breast cancer. Compositions of the instant invention may also be used to induce menses, to induce labor and for contraception.

### BRIEF DESCRIPTION OF THE DRAWINGS.

Fig. 1 illustrates a comparison of the Cmax (peak serum concentration) and area under the curve (AUC) following oral and vaginal administration of CDB-4124 or CDB-4453 at a 25 mg dose in beagles.
Fig. 2 illustrates the actual Cmax observed for Proellex (CDB-4124) and its mono-demethylated metabolite CDB-4453, after oral administration of CDB-4124 at 12.5 mg, 25 mg and 50 mg doses as well as the projected Cmax for 3 mg, 6 mg and 9 mg doses. Fig. 2 also illustrates the actual Cmax observed for Proellex (CDB-4124) and its mono-demethylated metabolite CDB-4453, after vaginal administration of CDB-4124 at 12.5 mg, 25 mg and 50 mg doses.
Fig. 3 illustrates a comparison of the inhibition of progesterone-induced endometrial proliferation in estradiol-primed immature rabbits following subcutaneous injection and oral administration of CDB-4124
Fig. 4 compares the antiprogestational effects of three doses of CDB-4124 when delivered orally versus when delivered to the vaginal mucosa of estradiol-primed immature rabbits in the presence of progesterone, as measured by a decrease in the McPhail index. Treatment with progesterone alone (vehicle control) provided a baseline measurement of progestational activity.

### DETAILED DESCRIPTION OF THE INVENTION

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any way. Embodiments illustrated under any heading may be combined with embodiments illustrated under any other heading.

It is to be understood that any ranges, ratios and ranges of ratios that can be formed by any of the numbers or data present herein represent further embodiments of the present invention. This includes ranges that can be formed that do or do not include a finite upper and/or lower boundary. Accordingly, the skilled person will appreciate that many such ratios, ranges and ranges of ratios can be unambiguously derived from the data and numbers presented herein and all represent embodiments of the invention.

Before the present compounds, compositions and methods are disclosed and described, it is to be understood that the terminology used herein is for the purpose of describing particular embodiments only and is not intended to be limiting. It must be noted that, as used in the present specification and the appended claims, the singular forms "a," "an" and "the" include plural referents unless the context clearly dictates otherwise.

### Definitions

The term "oral" administration means that the active agent is in a formulation designed to be ingested, i.e., designed to be delivered to the gastrointestinal system for absorption.

The term "effective dosage" means an amount of the composition's active component sufficient to treat a particular condition.

The term "selective progesterone receptor modulators" means compounds that affect functions of progesterone receptor in a tissue-specific manner. The compounds act as progesterone receptor antagonists in some tissues (for example, in breast tissue) and as progesterone receptor agonists in other tissues (for example, in the uterus).

The term "treat" or "treatment" as used herein refers to any treatment of any progesterone-dependent disorder or disease, and includes, but is not limited to, inhibiting the disorder or disease arresting the development of the disorder or disease; relieving the disorder or disease, for example, causing regression of the disorder or disease; or relieving the condition caused by the disease or disorder, relieving the symptoms of the disease or disorder.

The term "prevent" or "prevention," in relation to a progesterone-dependent disorder or disease, means preventing the onset of disorder or disease development if none had occurred, or preventing further disorder or disease development if the disorder or disease was already present. For example, compositions of the present invention may be used to prevent the recurrence of tumors. Recurrence of tumors may occur because of residual microscopic groups or nests of tumor cells which subsequently expand into clinically detectable tumors.

The term "progesterone agonist" means a compound that binds to a progesterone receptor and mimics the action of the natural hormone.

The term "progesterone antagonist" means a compound that binds to a progesterone receptor and inhibits the effect of progesterone.

The term "not substantially reduced" as used herein in reference to hormone levels in a female means that hormone levels are maintained within the normal range during administration of compositions of the invention. Thus, it is considered that some reduction in a hormone level may occur so long as the hormone level is maintained within the normal range.

The term "not substantially increased" as used herein in reference to hormone levels in a female means that hormone levels are maintained within the normal range during administration of compositions of the instant invention. Thus, it is considered that some elevation in a hormone level may occur so long as the hormone level is maintained within the normal range.

### Compounds

In one aspect, the present invention provides compounds having the general formula: 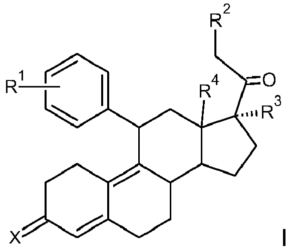 and pharmaceutically acceptable salts thereof. In Formula I, R¹ may be at the para, ortho or meta position and is a functional group including, but not limited to, -CH(OH)CH₃; alkyl; alkenyl; cycloalkyl; cycloalkenyl; aryl; H; CH₃SO; CH₃SO₂; acyl (e.g. formyl, acetyl, propionyl, butyryl and the like); alkoxy (e.g. -OCH₃, -O(CH₂)₂CH₃, -O-CH₂-CH=CH₂); thioalkoxy; thioalkyl (-SCH₃), acyloxy (e.g. acetoxy, propanoyloxy); Si(CH₃)₃; 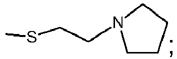 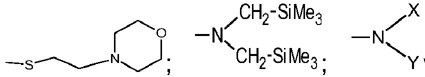 wherein X and Y are acyl; and a heterocyle preferably containing at least one nitrogen atom (e.g. aziridinyl 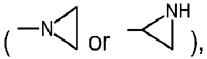 azirinyl 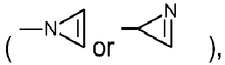 azetidinyl, pyrrolidinyl (-NC₄H₈), substituted pyrrolidinyl (e.g. methoxypyrrolidinyl, ethoxypyrrolidinyl), pyrrole 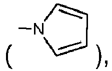 piperidinyl (-NC₅H₁₀), substituted piperidinyl (e.g., -O(CH₂)₂NC₅H₁₀), pyridinyl 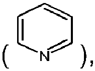 morpholinyl (NC₄H₈O), substituted morpholinyl (e.g., ethoxymorpholinyl), oxazinyl, piperazinyl 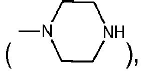 substituted piperazinyl (e.g., 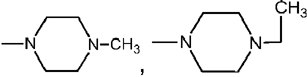 ), diazinyl, and an azole such as pyrazole 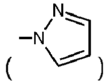 ). R² is a functional group including, but not limited to, hydrogen, halogen, alkyl, acyl, hydroxyl, akoxy (e.g., methoxy, ethoxy, vinyloxy, ethynyloxy, cyclopropyloxy, etc.), acyloxy (e.g., formyloxy, acetoxy, propionyloxy, heptanoyloxy, glycinate, etc.), alkyl carbonate, cypionyloxy, S-alkyl, S-CN, S-acyl and -OC(O)R⁶ wherein R⁶ is functional group including alkyl, alkoxyalkyl (e.g., -CH₂OCH₃) or alkoxy (-OCH₃). R³ is a functional group including but not limited to alkyl (e.g., methyl, methoxymethyl), hydroxy, alkoxy (e.g., methoxy, ethoxy, methoxymethyl, etc.), and acyloxy with the proviso that R³ is other than acetoxy or propynyl. R⁴ is a functional group including but not limited to hydrogen and alkyl. Finally, X is a functional group including but not limited to =O, =N-OR5 wherein R5 is hydrogen or alkyl, OH, CH₂, OAlk₁, and OCOAlk₂, wherein Alk₁ and Alk₂ are C1-C8 alkyl or C7-C15 aralalkyl, with the proviso that if R¹ is at the para position and is -OCH₃, -SCH₃, - NC₄H₈, -NC₅H₁₀, -NC₄H₈O, -CHO, -CH(OH)CH₃, -COCH₃, -O(CH₂)₂NC₄H₈, or -O(CH₂)₂NC₅H₁₀, X is other than =O or =N-OR5 wherein R5 is hydrogen or alkyl.

In one preferred embodiment, a compound of general formula I or a pharmaceutically acceptable salt thereof is provided wherein: R¹ is at the para position and is -OCH₃, -SCH₃, -NC₄H₈ (pyrrolidino), -NC₅H₁₀ (piperidino), -NC₄H₈O (morpholino), -CHO, -CH(OH)CH₃, -COCH₃, -O(CH₂)₂NC₄H₈ (methoxypyrrolidino) or -O(CH₂)₂NC₅H₁₀ (ethoxypiperidinophenyl); R² is hydrogen, halogen, alkyl, acyl, hydroxyl, akoxy (e.g., methoxy, ethoxy, vinyloxy, ethynyloxy, cyclopropyloxy, etc.), acyloxy (e.g. formyloxy, acetoxy, propionyloxy, heptanoyloxy, glycinate, etc.), alkyl carbonate, cypionyloxy, S-alkyl, S-CN, S-acyl and -OC(O)R⁶ wherein R⁶ is functional group including alkyl, alkoxyalkyl (e.g. - CH₂OCH₃) or alkoxy (-OCH₃); R³ is alkyl (e.g. methyl, methoxymethyl), hydroxy, alkoxy (e.g. methoxy, ethoxy, methoxymethyl, etc.), or acyloxy with the proviso that R³ is other than acetoxy or propynyl; R⁴ is hydrogen or alkyl; and X is OH, CH₂, OAlk1, or OCOAlk2, wherein Alk1 and Alk2 are C1-C8 alkyl or C7-C15 aralalkyl.

In another preferred embodiment, a compound of general formula I or a pharmaceutically acceptable salt thereof is provided wherein R¹ is at the meta or ortho position and is -OCH₃, -SCH₃, -NC₄H₈ (pyrrolidino), -NC₅H₁₀ (piperidino), -NC₄H₈O (morpholino), -CHO, -CH(OH)CH₃, -COCH₃, -O(CH₂)₂NC₄H₈ (methoxypyrrolidino) or -O(CH₂)₂NC₅H₁₀ (ethoxy-piperidinophenyl); R² is hydrogen, halogen, alkyl, acyl, hydroxyl, akoxy (e.g. methoxy, ethoxy, vinyloxy, ethynyloxy, cyclopropyloxy, etc.), acyloxy (e.g. formyloxy, acetoxy, propionyloxy, heptanoyloxy, glycinate, etc.), alkyl carbonate, cypionyloxy, S-alkyl, S-CN, S-acyl and -OC(O)R⁶ wherein R⁶ is functional group including alkyl, alkoxyalkyl (e.g. -CH₂OCH₃) or alkoxy (-OCH₃); R³ is alkyl (e.g. methyl, methoxymethyl), hydroxy, alkoxy (e.g. methoxy, ethoxy, methoxymethyl, etc), or acyloxy with the proviso that R³ is other than acetoxy or propynyl; R⁴ is hydrogen or alkyl; and X is =O, =N-OR5 wherein R5 is hydrogen or alkyl, OH, CH₂, OAlk1, or OCOAlk2, wherein Alk1 and Alk2 are C1-C8 alkyl or C7-C15 aralalkyl.

In yet another preferred embodiment, a compound of general formula I or a pharmaceutically acceptable salt thereof is provided wherein R¹ is at the para position and is alkyl; alkenyl; cycloalkyl; cycloalkenyl; aryl; H; CH3SO; CH3SO2; thioalkoxy; Si(CH₃)₃; 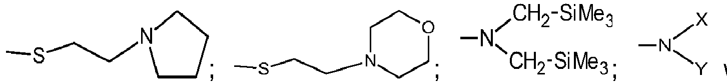 wherein X and Y are acyl; aziridinyl, azirinyl, azetidinyl, methoxypyrrolidinyl, ethoxymorpholinyl, oxazinyl, piperazinyl, methylpiperazinyl, ethylpiperazinyl or diazinyl; R² is hydrogen, halogen, alkyl, acyl, hydroxyl, akoxy (e.g. methoxy, ethoxy, vinyloxy, ethynyloxy, cyclopropyloxy, etc.), acyloxy (e.g. formyloxy, acetoxy, propionyloxy, heptanoyloxy, glycinate, etc.), alkyl carbonate, cypionyloxy, S-alkyl, S-CN, S-acyl or -OC(O)R⁶ wherein R⁶ is functional group including alkyl, alkoxyalkyl (e.g., -CH₂OCH₃) or alkoxy (-OCH₃); R³ is alkyl (e.g. methyl, methoxymethyl), hydroxy, alkoxy (e.g. methoxy, ethoxy, methoxymethyl, etc), or acyloxy with the proviso that R³ is other than acetoxy or propynyl; R⁴ is hydrogen or alkyl; and X is =O, =N-OR5 wherein R5 is hydrogen or alkyl, OH, CH₂, OAlk1, or OCOAlk₂, wherein Alk1 and Alk2 are C1-C8 alkyl or C7-C15 aralalkyl.

In yet another preferred embodiment, a compound of general formula I or a pharmaceutically acceptable salt thereof is provided wherein R¹ is at the meta or ortho position and is is alkyl; alkenyl; cycloalkyl; cycloalkenyl; aryl; H; CH3SO; CH3SO2; thioalkoxy; Si(CH₃)₃; 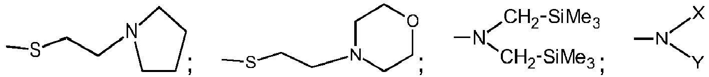 wherein X and Y are acyl; aziridinyl, azirinyl, azetidinyl, methoxypyrrolidinyl, ethoxymorpholinyl, oxazinyl, piperazinyl, methylpiperazinyl, ethylpiperazinyl or diazinyl; R² is hydrogen, halogen, alkyl, acyl, hydroxyl, akoxy (e.g. methoxy, ethoxy, vinyloxy, ethynyloxy, cyclopropyloxy, etc.), acyloxy (e.g. formyloxy, acetoxy, propionyloxy, heptanoyloxy, glycinate, etc.), alkyl carbonate, cypionyloxy, S-alkyl, S-CN, S-acyl or -OC(O)R⁶ wherein R⁶ is functional group including alkyl, alkoxyalkyl (e.g., -CH₂OCH₃) or alkoxy (-OCH₃); R³ is alkyl (e.g., methyl, methoxymethyl), hydroxy, alkoxy (e.g., methoxy, ethoxy, methoxymethyl, etc.), or acyloxy with the proviso that R³ is other than acetoxy or propynyl; R⁴ is hydrogen or alkyl; and X is =O, =N-OR5 wherein R5 is hydrogen or alkyl, OH, CH₂, OAlk1, or OCOAlk2, wherein Alk1 and Alk2 are C1-C8 alkyl or C7-C15 aralalkyl.

Particularly preferred R¹ substituents, whether at the ortho, meta or para position, are -CHO, -COCH₃ and heterocyle preferably containing at least one nitrogen atom, particularly -NC₅H₁₀ (piperidino).

Particularly preferred R² substituents are alkoxy (particularly methoxy or ethoxy) and H.

Particularly preferred R³ substituents are alkoxy (particularly methoxy or ethoxy), propionyloxy, formyloxy, and methoxymethyl.

Particularly preferred R⁴ substituents are alkyls, preferably methyl.

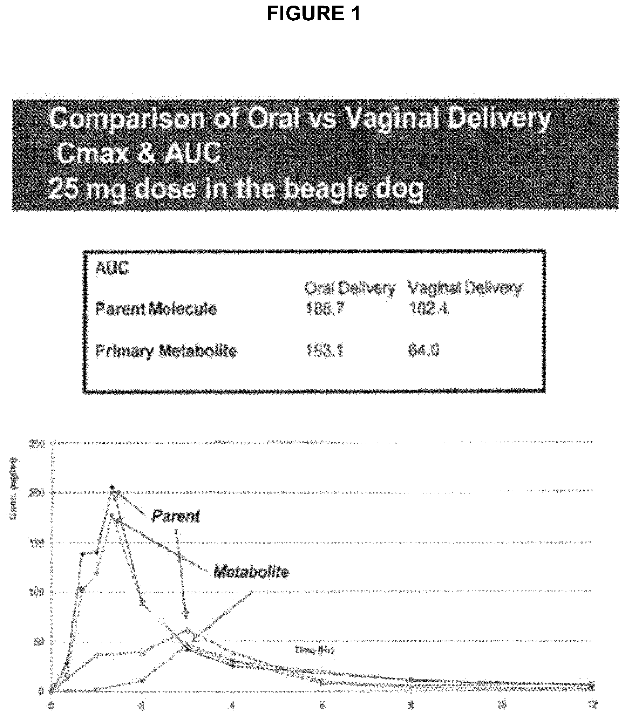

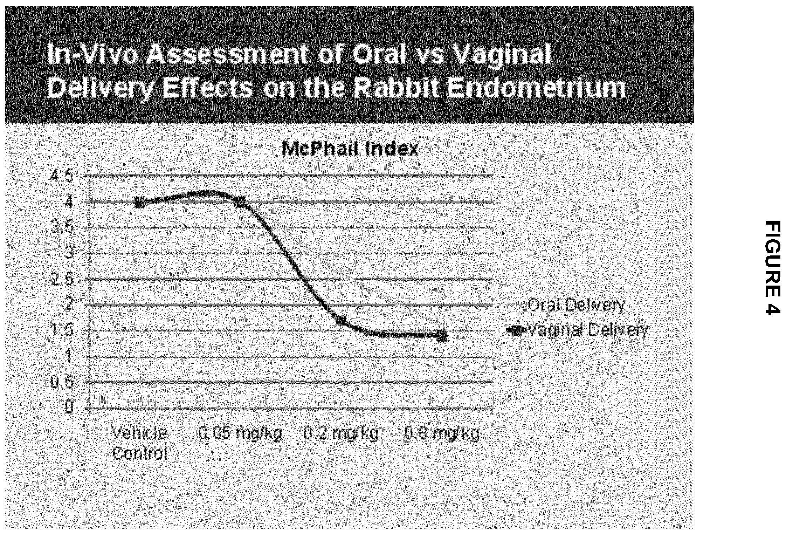

The compounds of general formula I can be synthesized by conventional synthetic chemistry techniques, including those used to synthesize the compounds disclosed in US Patent Nos. 6,861,415 and 6,900,193, the contents of each of which are hereby incorporated by reference. In particular, the synthetic schemes set forth in Figures 1, 2 and 3 of US Patent No. 6,861,415 and Figures 1-11 of US Patent No. 6,900,193 may be used, in combination with synthetic techniques known in the art, to synthesize compounds of the invention.

Compounds of general formula I possess a phenyl group at C11β, which is substituted at the ortho, meta or para position (i.e., at position R¹ of general formula I) with a functional group that cannot be metabolized to produce a primary amine upon administration of the compound. For example, compounds having a dimethylaminophenyl group at the C11β position undergo dealkylation upon administration to yield the primary amine aniline (-phenyl-NH₂) at the C11β position. The dealkylation occurs in two steps: first, the dimethylaminophenyl group is monodemethylated relatively quickly to monomethylaminophenyl; second, in a relatively slow reaction, the remaining alkyl group is removed to form the primary amine. Without being bound by theory, it is believed that aniline or substituted aniline (phenyl-NRH) groups may serve as reactive nucleophiles contributing to adverse liver reactions in patients who have received these compounds by the formation of protein adducts, particularly when administered long term at relatively high doses. Accordingly, R¹ is not a primary, secondary or tertiary amine. Moreover, R¹ is not a functional group other than a primary, secondary or tertiary amine which is itself substituted with a primary, secondary or tertiary amine. The compounds of the present invention are therefore unexpectedly useful for the long-term treatment of hormone-dependent disorders.

The compounds of the general formula I also have other than acetoxy or propynyl at the C17α position (i.e., position R³ of general formula I). Without being bound by theory it is believed that these moieties, when present at the C17α position, may be metabolized upon administration to an alcohol which may contribute to the formation of protein adducts and liver toxicity in patients who have been administered such compounds.

In a related embodiment, the present invention relates to methods of treating a progesterone-dependent condition by administering one or more compounds of general formula I (or a pharmaceutical composition comprising one or more compounds of general formula I) as described above. Compounds of general Formula I are not expected to contribute to adverse liver reactions in patients who have received these compounds and therefore, according to this aspect of the present invention, may be administered through any route, including without limitation oral (i.e., administration to the gastrointestinal tract), sublingual/buccal, intravascular, intramuscular, subcutaneous, inhalation, mucosal (e.g., rectal or vaginal), and topical. In a preferred embodiment, a composition comprising one or more compounds of general formula I is administered orally at a dosage of at least 25 mg/day, preferably at least 50 mg/day, to treat a hormone-dependent condition for a period of at least 2, 3, 4, 5, 6, 7, 8, 9, 10 or more months.

### Methods

Also provided by the present invention are methods of administering antiprogestins for the treatment of hormone (e.g., progesterone) dependent conditions which avoid liver toxicity.

In one embodiment, the present invention relates to methods of treating a progesterone-dependent condition by oral administration of any steroid antiprogestin having a C11β substituent other than an amino group, an N-monoalkylamino group, an N-dialkylamino group or a functional group substituted with an amino, N-monoalkylamino, or N-dialkylamino group. For example, the C11β substituent of the steroid antiprogestin is other than N,N-dialkylaminoaryl, N-monoalkylaminoaryl and aminoaryl. The steroid antiprogestin having a C11β substituent other than an amino group, an N-monoalkylamino group, an N-dialkylamino group or a functional group substituted with an amino, N-monoalkylamino, or N-dialkylamino group may be administered orally at a dosage of at least 25 mg/day, more preferably at least 50 mg/day for a period of at least 2, 3, 4, 5, 6, 7, 8, 9, 10 or more months. Representative steroid anti-progestins having a C11β substituent other than an amino group, an N-monoalkylamino group, an N-dialkylamino group or a functional group substituted with an amino, N-monoalkylamino, or N-dialkylamino group include CDB-4119 (17α-acetoxy-11β-(4-acetylphenyl)-21-thioacetoxy-19-norpregna-4,9-diene-3,20-dione), CDB-4239 (17α-acetoxy-11β-(4-acetylphenyl)-21-methoxy-19-norpregna-4,9-diene-3,20-dione), CDB-4241 (17α,21-diacetoxy-11β-(4-acetyiphenyl)-19-nor-pregna-4,9-diene-3,20-dione), CDB-4176 (17α-acetoxy-11β-(4-acetylphenyl)-19-norpregna-4,9-diene-3,20-dione), CDB-4363 (17α-acetoxy-11β-(4(N-piperidino)phenyl)-19-norpregna-4,9-diene-3,20-dione) and other compounds disclosed in U.S. Patent Nos. 6,861,415 and 6,900,193.

In another embodiment, the present invention relates to non-oral administration of a composition comprising one or more antiprogestins to treat a hormone (e.g., progesterone) dependent condition. Antiprogestins according to this aspect of the invention may be any known antiprogestin including compounds of general Formula I described above. This aspect of the invention arises in part from the unexpected finding that certain 19-nortestosterone- or 19-norprogesterone-derived antiprogestins can exhibit toxic effects on the liver at therapeutic concentrations, limiting their clinical use. Specifically, it has been found that patients subjected to chronic daily administration of therapeutic oral (i.e., for ingestion) dosages of the antiprogestin/SPRM CDB-4124 exhibit liver toxicity. Large amounts of the mono-demethylated metabolite of CDB-4124 are detected by pharmacokinetic studies on patients subsequent to oral ingestion of CDB-4124, indicating CDB-4124 undergoes significant first pass metabolism in the liver providing the opportunity for liver damage.

In a preferred embodiment, the present invention relates to non-oral administration of a composition comprising a steroid antiprogestin having a C11β substituent other than an amino group, an N-monoalkylamino group, an N-dialkylamino group or a functional group substituted with an amino, N-monoalkylamino, or N-dialkylamino group, such as, without limitation, CDB-4119, CDB-4239, CDB-4241, CDB-4176, and CDB-4363. These compounds have C11β substituents which are not expected to form protein adducts in the liver and toxic liver effects are further avoided by circumventing first-pass metabolism by administering the compounds non-orally. In a related embodiment, the compounds are administered non-orally at a therapeutically effective dose that is relatively low compared to the therapeutically effective dose of the compound when administered orally. For example, when administered locally to the vaginal mucosa, the therapeutically effective dose may be less than 50 mg/day, less than 40 mg/day, less than 30 mg/day less than 20 mg/day, less than 10 mg/day, less than 5mg/day, between 5mg/day and 50mg/day, between 5mg/day and 40mg/day, between 5mg/day and 30mg/day, between 5mg/day and 20mg/day, or between 5mg/day and 10mg/day. In another related embodiment, the effective amount of the compound is less than the effective amount when administered systemically, for example, the effective amount when administered locally to the vaginal mucosa may be 2-fold, 3-fold, 4-fold 5-fold, 6-fold, 7-fold, 8-fold, 9-fold and even 10-fold less than the effective amount when administered systemically to treat endometriosis, uterine fibroids and other diseases located in that region.

Compounds of general Formula I as described above are expected to exhibit reduced or no liver toxicity whether delivered through an oral or non-oral route, making them suitable for use in treating various progesterone-dependent conditions when administered via any administration route including without limitation oral, sublingual/buccal, intravascular, intramuscular, subcutaneous, inhalation, mucosal (e.g., rectal or vaginal), and topical.

Non-oral administration of antiprogestins, including compounds of general formula I described above, may reduce liver toxicity compared to oral administration of the same compounds. Preferably, the antiprogestins are administered by a route which avoids first pass metabolism such as, without limitation, intravenous, intramuscular, sublingual and mucusoal (e.g., vaginal, intrauterine or rectal).

The antiprogestin may be any compound which inhibits the progesterone receptor such as a specific progesterone receptor modulator (SPRM), so long as the antiprogestin has low glucorticoid activity. Preferably, the antiprogestin has low estrogenic/antiestrogenic activity such that serum estrogen levels are substantially preserved in the patient following administration of the antiprogestin.

With respect to the various embodiments described below, compositions of the invention may comprise one or more compounds of general Formula I described above in which case the composition may be administered orally or non-orally. Alternatively, the composition may comprise any antiprogestin other than those of general Formula I described above in which case the composition is administered by a route which avoids first pass metabolism of the antiprogestin.

In one embodiment of the invention, a composition of the invention is administered to a patient with breast cancer in order to treat the breast cancer. In a preferred embodiment, the patient is a human female and the breast cancer expresses human estrogen receptor (hER) or human progesterone receptor (hPR) and more preferably expresses both hER and hPR.

In a related embodiment of the invention, a composition of the invention is administered to a breast cancer patient with one or more tumors resistant to antiestrogen treatments in order to treat the breast cancer. For example, compounds of the instant invention may be particularly useful for treating tamoxifen-resistant breast cancer in patients.

In a related embodiment of the invention, a composition of the invention is administered to a patient suffering from a disorder selected from the group consisting of ductal carcinoma in situ (DCIS), mucinous (colloid) carcinoma, medullary carcinoma of the breast, papillary carcino-ma of the breast, adenoid cystic carcinoma (ACC), Paget's disease of the nipple, inflammatory breast disease, fibroadenoma and fibrocystic breast disease in order to treat the disorder.

In another embodiment of the invention, a composition of the instant invention is administered to a female undergoing estrogen therapy in order to prevent the development of breast cancer in the female.

In a related embodiment, the composition is administered by a (non-oral) route that avoids first pass metabolism selected from the group consisting of: sublingual/buccal, intravascular, intramuscular, subcutaneous, inhalation, mucosal (e.g., rectal, intrauterine or vaginal), and topical. In a preferred embodiment, a composition of the invention is administered to a breast cancer patient in the form of a trans-dermal patch, gel or ointment that is applied directly to the breast (e.g., to the nipple or areola) in order to treat the breast cancer.

In another embodiment of the invention, a composition of the invention is administered to a female patient in need thereof in order to suppress endometrial proliferation. In a preferred embodiment, a composition of the invention is vaginally administered to a patient in order to suppress endometrial proliferation.

In a related embodiment of the invention, a composition of the invention is administered to a female patient in need thereof in order to treat endometriosis. In a preferred embodiment, a composition of the invention is vaginally administered to a patient in order to treat endometriosis.

In another embodiment of the invention, a composition of the invention is administered to a female in need thereof in order to treat dysmenorrhea. In a preferred embodiment, a composition of the invention is vaginally administered to a patient in order to treat dysmenorrhea.

In yet another embodiment of the invention, a composition of the invention is administered to a female in need thereof in order to treat uterine fibroids. In a preferred embodiment, a composition of the invention is vaginally administered to a patient in order to treat uterine fibroids.

In another embodiment of the invention, a composition of the invention is administered to a female patient in need thereof in order to treat adenomyosis. In a preferred embodiment, a composition of the invention is vaginally administered to a patient in order to treat adenomyosis.

In another embodiment of the invention, a composition of the invention is administered to a female patient in need thereof in order to treat an endometrioma. In a preferred embodiment, a composition of the invention is vaginally administered to a patient in order to treat an endometrioma.

In another embodiment of the invention, a composition of the invention is administered to a female patient in need thereof in order to treat ovarian cancer. In a preferred embodiment, a composition of the invention is vaginally administered to a patient to treat ovarian cancer.

In another embodiment of the invention, a composition of the invention is administered to a female patient in need thereof in order to treat cervical cancer. In a preferred embodiment, a composition of the invention is vaginally administered to a patient to treat cervical cancer.

In a particularly preferred embodiment, a composition of the invention is administered to a patient suffering from endometriosis, dysmennorrhea, uterine fibroids, adenomyosis, ovarian cancer or cervical cancer by a non-oral administration route designed to provide local delivery of the antiprogestin to the affected region. The antiprogestin may be formulated into a suitable preparation for such non-oral local administration. For example, the antiprogestin may be formulated, without limitation, as a depot injection (e.g. solid or oilbased subcutaneous or intramuscular) designed to slowly release the antiprogestin over a long period of time; an intravaginal preparation such as a doughnut-shaped hormone-releasing vaginal ring; a vaginal suppository; a vaginal pill; an intra-uterine preparation such as an intrauterine device (IUD) or matrix preparation; an implantable drug delivery device; a topical gel; or a trans-dermal patch. Preferably, the antiprogestin is incorporated into a vaginal ring, uterine depot, vaginal suppository or the like which maintains a slow but continual release of the antiprogestin that is locally but not systemically significant.

In a preferred embodiment, endometriosis, dysmennorhea, uterine fibroids, adenomyosis, ovarian cancer or cervical cancer is treated by administering an intravaginal preparation containing an antiprogestin to the vagina of a patient in need of such treating. It is understood that the antiprogestin is absorbed from the vaginal mucosa that is in direct contact with the intravaginal preparation. An intravaginal ring is a preferred intravaginal preparation and can be designed to provide continuous release of the antiprogestin in the vagina. The insertion period may be, e.g., from 1 to 3 months after which the preparation may be replaced by a new preparation to provide a continuous long-term treatment.

In another preferred embodiment, endometriosis, dysmennorhea, uterine fibroids, adenomyosis, ovarian cancer or cervical cancer is treated by administering a vaginal pill or vaginal suppository containing an antiprogestin to the vagina of a patient in need of such treating. The vaginal pill and vaginal suppository can be produced by well-known methods using additives such as a diluting agent, a binding agent and a suppository base that are commonly used in the production of such preparations.

In another preferred embodiment, endometriosis, dysmennorhea, uterine fibroids, adenomyosis, ovarian cancer or cervical cancer is treated by administering an intrauterine preparationcontaining an antiprogestin to the uterine cavity of a patient in need of such treating. The intrauterine preparation may be a matrix preparation which provides continuous release of the antiprogestin in the uterus. The insertion period of the intrauterine preparation may be about 6 months, after which the preparation may be removed and a new preparation inserted so that long term treatment of the disorder is achieved. The intrauterine preparation may be produced by routine methods using a matrix base (e.g., a polymer including but not limited to a silicon rubber, ethylene vinyl acetate, ethyl cellulose, carboxymethylethylcellulose, polyethylene glycol, polyvinyl alcohol, carboxyvinyl polymer or collagen) an inert intrauterine device and optionally an appropriate crosslinking agent and/or release promoting agent such as polysorbate 60, polysorbate 80, glycerin, isopropyl palmitate and isopropyl myristate. The matrix preparation may be single-layered or two-layered. The form of the intrauterine preparation is not limited but is sufficient to have suitable form for topical administration in the uterus.

In another embodiment of the invention, a composition of the invention is administered to female in need thereof in order to induce menses in the female.

In yet another embodiment of the invention, a composition of the invention is administered to a female in need thereof in order to induce labor.

In yet another embodiment of the invention, a composition of the invention is administered to female in need thereof as a contraceptive.

Compositions comprising a compound of General Formula I, as described above, may be suitable for prolonged oral administration because these compounds are expected to exhibit reduced or no liver toxicity. Alternately, antiprogestins (e.g., compounds of General Formula I) may be chronically administered by a route that avoids first pass metabolism and therefore reduces or eliminates metabolism by the liver. Thus, compositions of the invention may be administered on a chronic basis without causing toxic liver effects. Preferably, the compounds have only low glucocorticoid receptor binding activity and therefore do not interfere with functions of glucocorticoid receptor. Thus, compositions of the invention may also be associated with reduced side effects such as mood swings, fatigue and weight loss, typically found when antiprogestins with a high affinity for glucocorticoid receptor are used. Preferably, compounds of the instant invention also have low, or substantially no, estrogenic, anti-estrogenic and anti-androgenic activities.

In one embodiment, a composition of the invention comprising one or more antiprogestins in an amount effective for treating a hormone dependent condition is administered for an administration period of least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 or more days. The composition may also be administered for an administration period of least 1, 2, 3, 4, 5, 6, 7, 8, 9, 10, 11, 12 or more months. The composition may also be administered for an administration period of at least 1, 2, 3, 4, 5, 6, 7, 8, 9, 10 or more years. During the administration period, the composition may be administered daily or periodically such as every other day, every other month, and the like. The composition may also be administered intermittently. For example, the composition may be administered for an administration period of 1, 2, 3, 4, 5 or more months, followed by a period of discontinuance, followed by an administration period of 1, 2, 3, 4, 5 or more months, and so on.

In one embodiment, the composition is administered intermittently such that the subject undergoes menses during at least one discontinuance period. This approach is expected to avoid the adverse effects associated with a thickened or stagnant endometrium that may accompany extended treatment with progesterone antagonists, such as spotting, breakthrough bleeding, endometrial hyperproliferation or endometrial cancer. At least one, and preferably every discontinuance period is of sufficient length for the subject to experience menstruation. More preferably, the subject experiences menstruation during every discontinuance period. In a particularly preferred embodiment, the composition is administered daily for an administration period of four months, followed by a discontinuance period during which the subject experiences menstruation, followed by another administration period of four months, and so on.

In one embodiment, any of the steroid compounds disclosed in U.S. Patent Nos. 6,861,415 and 6,900,193 may be administered to a patient by a route that avoids first pass metabolism. In a preferred embodiment, the steroid compound is CDB-4124 (21-methoxy-17a-acetoxy-11β-(4-N,N-dimethylaminophenyl)-19-norpregna-4,9-diene-3,20-dione) having the following structural formula:

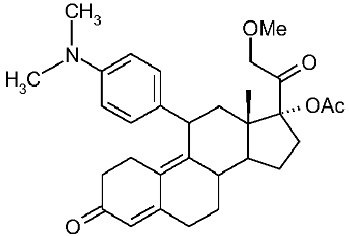

CDB-4453 (21-methoxy-17α-acetoxy-11β-(4-N-methylaminophenyl)-19-norpregna-4,9-diene-3,20-dione), a monodemethylated derivative of CDB-4124, has been demonstrated to possess even lower anti-glucocorticoid activity than its parent. Attardi et al., 2002, Mol. Cell. Endocrin. 188:111-123, the contents of which are incorporated herein by reference. Accordingly, 19-nortestosterone or 19-norprogesterone-derived antiprogestins with a monomethylamine-substituted phenyl ring at the 11β-position of carbon 11, such as CDB-4453, are one type of preferred compound for use in the methods of the invention; however, because these compounds, and their di-demethylated metabolites, have surprisingly been found to cause toxic liver effects when administered orally, these compounds are to be administered through a route that avoids first pass metabolism. For example, the compound 21-methoxy-11β-(4-N-methylaminophenyl)-19-norpregna-4,9-diene-3,20-dione is a preferred compound when administered by a route that avoids first pass metabolism as are compounds described in US Patent Numbers 6,861,415 and 6,900,193 wherein R¹ is -NHCH₃.

Other compounds useful according to the present methods when administered by a route that avoids first pass metabolism include, without limitation, the following:

Mifepristone (RU-486; 11β-[4-N,N-dimethylaminophenyl]-17β-hydroxy-17-(1-propynyl)-estra-4,9-dien-3-one), Lilopristone (11β-(4 N,N-dimethylaminophenyl)-17β-hydroxy-17-((Z)-3-hydroxypropenyl)estra-4,9-dien-3-one), Onapristone (11β-(4 N,N-dimethylaminophenyl)-17α-hydroxy-17-(3-hydroxypropyl)-13α-estra-4,9-dien-3-one), asoprisnil (benzaldehyde, 4-[(11β,17β)-17-methoxy-17-(methoxymethyl)-3-oxoestra-4,9-dien-11-yl]-1-(E)-oxim; J867), its metabolite J912 (4-[17β-Hydroxy-17α-(methoxymethyl)-3-oxoestra-4,9-dien-11β-yl]benzaldehyd-(1E)-oxim), and other compounds described in DE 43 32 283 and DE 43 32 284, and CDB-2914 (17α-acetoxy-11β-(4-N,N-dimethylaminophenyl)-19-norpregna-4,9-dien-3,20-dione) and other compounds described in Stratton et al., 2000, Hu. Reprod. 15:1092-1099.

Also contemplated for use in the present invention are JNJ-1250132 and other compounds described in Allan et al., 2006, Steroids 71:949-954; 5-Aryl-1,2-dihydro-chromeno[3,4-f]quinolines described in Zhi et al., 1998, J. Med. Chem. 41:291-302; 1,4-dihydro-benzo[d][1,3]oxazin-2-ones described in U.S. Patent Nos.: 6,509,334, 6,566,358 and 6,713,478 to Zhang et al.; 1,3-dihydro-indol-2-ones described in U.S. Patent No. 6,391,907 to Fensome et al.*;* 2,3-dihydro-1H-indoles described in U.S. Patent No. 6,417,214 to Ulrich *et* a/.; benzimidazolones and analogues thereof described in U.S. Patent No. 6,380,235 to Zhang *et a*/*.;* 2,1-benzisothiazoline 2,2-dioxides described in U.S. Patent No. 6,339,098 to Collins *et a*/*.;* cyclocarbamates and cyclo-amides described in U.S. Patent Nos.: 6,306,851 and 6,441,019 to Santilli *et a*/*.;* cyclic urea and cyclic amide derivatives described in U.S. Patent No. 6,369,056 to Zhang *et a*/*.;* and quinazolinone and benzoxazine derivatives described in U.S. Patent No. 6,358,948 to Zhang et al.

Other antiprogestins that may be useful in the invention include, without limitation, (6a,11β,17β)-11-(4-dimethylaminophenyl)-6-methyl-4',5'-dihydrospiro[estra-4,9-diene-17,2'(3'H)-furan]-3-one (ORG-31710) and other compounds described in U.S. Patent No. 4,871,724; (11β,17α)-11-(4-acetylphenyl)-17,23-epoxy-19,24-dinorchola-4,9,20-trien-3-one (ORG-33628); (7β,11β,17β)-11-(4-dimethylaminophenyl-7-methyl]-4',5'-dihydrospiro[estra-4,9-diene-17,2'(3'H)-furan]-3-one (ORG-31806) and other compounds described in U.S. Patent No. 4,921,845; ZK-112993 and other compounds described in Michna et al., 1992, J. Steroid Biochem. Molec. Biol. 41:339-348; ORG-31376; ORG-33245; ORG-31167; ORG-31343; RU-2992; RU-1479; RU-25056; RU-49295; RU-46556; RU-26819; LG1127; LG120753; LG120830; LG1447; LG121046; CGP-19984A; RTI-3021-012; RTI-3021-022; RTI-3021-020; RWJ-25333; ZK-136796; ZK-114043; ZK-230211; ZK-136798; ZK-98229; ZK-98734; and ZK-137316.

Further contemplated for use in the present invention are the 11β-aryl-4-estrenes such as (Z)-11β-[(4-Dimethylamino)phenyl)]-17β-hydroxy-17α-(3-hydroxy-1-propenyl)estr-4-en-3-one described in U.S. Patent No. 5,728,689; the 11β-aryl-estrene derivatives described in U.S. Patent Nos. 5,843,933 and 5,843,931; the 11-benzaldoxime-estra-diene derivatives such as 4-[17β-Methoxy-17α-(methoxymethyl)-3-oxoestra-4,9-dien-11β-yl]benzaldehyde-1-(E)-oxime described in U.S. Patent No. 5,693,628; the 11-benzaldoxime-17β-methoxy-17α-methoxymethyl-estradiene derivatives such as 4-[17β-Methoxy-17α-(methoxymethyl)-3-oxoestra-4,9-dien-11β-yl]benzaldehyde-1-(E)-[O-(ethylamino)carbonyl]oxime described in U.S. Patent No. 5,576,310; the S-substituted 11β-benzadoxime-estra-4,9-diene-carbonic acid thioesters such as 4-[17β-Methoxy-17α-(methoxymethyl)-3-oxoestra-4,9-dien-11β-yl]benzaldehyde-1-(E)-[O-(ethyl-thio)carbonyl]oxime, described in WO 99/45023; the steroid esters such as (Z)-6'-(4-cyanophenyl)-9,11α-dihydro-17β-hydroxy-17α-[4-(1-oxo-3-methyl-butoxy)-1-butenyl]4'H-naphtho [3',2',1';10,9,11]estr-4-en-3-one described in DE 19652408, DE 4434488, DE 4216003, DE 4216004 and WO 98/24803; the fluorinated 17α-alkyl chain steroids such as 11β-(4-acetylphenyl)-17β-hydroxy-17α-(1,1,2,2,2-pentafluoroethyl)estra-4,9-dien-3-one described in WO 98/34947; the 17-spirofuran-3'-ylidene steroids such as 11β-(4-Acetylphenyl)-19,24-dinor-17,23-epoxy-17α-chola-4,9,20-trien-3-one described in U.S. Patent No. 5,292,878; (Z)-11β,19-[4-(3-Pyridinyl)-o-phenylene]-17β-hydroxy-17α-[3-hydroxy-1-propenyl]-4-androsten-3-one and other compounds described in U.S. Patent No. 5,439,913; the 13-alkyl-11β-phenyl gonanes such as 11β-[4-(1-methylethenyl)phenyl]-17α-hydroxy-17β-(3-hydroxypropyl)-13α-estra-4,9-dien-3-one described in U.S. Patent No. 5,446,036; the 11-arylsteroids such as 4',5'-Dihydro-11β-[4-(dimethylamino)phenyl]-6β-methylspiro[estra-4,9-dien-17β,2'(3'H)-furan]-3-one described in U.S. Patent No. 4,921,845; the 11β-aryl-estradienes described in U.S. Patent Nos. 4,829,060, 4,814,327 and 5,089,488; the 11β-aryl-4,9 gonadiens and 11β-aryl-13-alkyl-4,9-gonadiens described in U.S. Patent Nos.: 5,739,125, 5,407,928 and 5,273,971; the 11β-aryl-6-alkyl (or alkenyl or alkinyl) steroids described in EP 289073; the 10β,11β-bridged steroids described in U.S. Patent No. 5,093,507; the 11β-aryl-14-β-steroids described in U.S. Patent No. 5,244,886; the 19,11β-bridged steroids described in U.S. Patent Nos: 5,095,129, 5,446,178, 5,478,956 and 5,232,915; the 1-arylsulphonyl, arylcarbonyl and 1-arylphosphonyl-3-phenyl-1,4,5,6-tetrahydro-pyridazines described in U.S. Patent No. 5,684,151; the 1-arylsulphonyl, arylcarbonyl and arylthiocarbonyl pyridazino derivatives described in U.S. Patent No. 5,753,655; the 1,2-dihydro-[1,2-g]quinoline derivatives and 1,2-dihydro-chromeno-[3,4-f]quinoline derivatives described in U.S. Patent Nos: 5,688,808,. 5,693,646, 5,693,647, 5,696,127, 5,696,130 and 5,696,133; the oxa-steroids 6 derived from (8S, 13S, 14R)-7-oxa-estra-4,9-diene-3,17-dione 1 described in Kang et al., 2007, Bioorg. Med. Chem. Lett. 15:907-910; and the 7-oxa-steroids 4 described in Kang et al., 2007, Bioorg. Med. Chem. Lett. 17:2531-2534.

Further contemplated for use in the present invention are the19-nor steroids and 19-nor-D-homo steroids having the general formulas disclosed in U.S. Patent Nos. 4,386,085, 4,447,424, 4,519,946 and 4,634,695, each of which is incorporated herein by reference in its entirety.

Further contemplated for use in the present invention are the 11β-aryl estradienes having formula I disclosed in U.S. Patent No. 4,536,401, the entire content of which is incorporated herein by reference.

Further contemplated for use in the present invention are the 13α-alkyl-gonanes having formula I disclosed in U.S. Patent No. 4,780,461, the entire content of which is incorporated herein by reference.

Further contemplated for use in the present invention are 11β-arylestradienes having general Formula I of U.S. Patent No. 4,609,651, the entire content of which is incorporated herein by reference.

Further contemplated for use in the present invention are 11β-aryl-4-estrenes of formula I of U.S. Patent No. 5,728,689, the entire content of which is incorporated herein by reference.

Further contemplated for use in the present invention are 11β-aryl-4-estrene derivatives of formula I of U.S. Patent Nos. 5,843,933 and 5,843,931, each of which is incorporated herein by reference in its entirety.

Further contemplated for use in the present invention are 11-arylsteroids disclosed in U.S. Patent No. 4,921,845, the entire content of which is incorporated herein by reference.

Further contemplated for use in the present invention are 11β-aryl-gona-4,9-dien-3-ones of formula I of U.S. Patent No. 5,739,125, the entire content of which is incorporated herein by reference.

Further contemplated for use in the present invention are 11β-aryl-gona-4,9-dienes of formula I of U.S. Patent No. 5,407,928, the entire content of which is incorporated herein by reference.

Further contemplated for use in the present invention are the oxa-steroids 6 compounds disclosed in Kang et al., Bioorg. Med. Chem. Lett., 17(4):907-910 (2007).

Antiprogestin compounds that may be employed in accordance with the present invention can be synthesized using synthetic chemistry techniques known in the art such as those disclosed in U.S. Patent No. 6,861,415. It is to be understood that certain functional groups may interfere with other reactants or reagents under the reaction conditions and therefore may need temporary protection. The use of protecting groups is described in 'Protective Groups in Organic Synthesis', 2nd edition, T. W. Greene & P. G. M. Wutz, Wiley-Interscience (1991).

In one embodiment, compositions of the invention comprise a pharmaceutically acceptable salt of an antiprogestin, for example a compound of general formula I as described above. Depending on the process conditions the salt compound obtained may be either in neutral or salt form. Salt forms include hydrates and other solvates and also crystalline polymorphs. Both the free base and the salts of these end products may be used in accordance with the invention.

Acid addition salts may in a manner known per se be transformed into the free base using basic agents such as alkali or by ion exchange. The free base obtained may also form salts with organic or inorganic acids.

In the preparation of acid addition salts, preferably such acids are used which form suitably pharmaceutically acceptable salts. Examples of such acids are hydrochloric acid, sulfuric acid, phosphoric acid, nitric acid, aliphatic acid, alicyclic carboxylic or sulfonic acids, such as formic acid, acetic acid, propionic acid, succinic acid, glycolic acid, lactic acid, malic acid, tartaric acid, citric acid, ascorbic acid, glucuronic acid, fumaric acid, maleic acid, hydroxymaleic acid, pyruvic acid, aspartic acid, glutamic acid, p-hydroxybenzoic acid, embonic acid, ethanesulfonic acid, hydroxyethanesulfonic acid, phenylacetic acid, mandelic acid, alogenbensenesulfonic acid, toluenesulfonic acid, galactaric acid, galacturonic acid or naphthalenesulfonic acid. All crystalline form polymorphs may be used in accordance with the invention.

Base addition salts may also be used in accordance with the invention and may be prepared by contacting the free acid form with a sufficient amount of the desired base to produce the salt in the conventional manner. The free acid form may be regenerated by contacting the salt form with an acid and isolating the free acid in the conventional manner. Pharmaceutically acceptable base addition salts are formed with metals or amines, such as alkali and alkali earth metals or organic amines. Examples of metals used as cations are sodium, potassium, calcium, magnesium and the like. Examples of suitable amines are amino acids such as lysine, choline, diethanolamine, ethylenediamine, N-methylglucamine and the like.

Compositions of the instant invention can be prepared in the form of a dose unit or dose units suitable for oral (only in the case of compounds of general formula I as described above), sublingual/buccal, parenteral, transdermal, transmucosal (e.g., vaginal or rectal), or topical administration. Parenteral administration includes, but is not limited to, intravenous, intraarterial, intraperitoneal, subcutaneous, intramuscular, intrathecal, and intraarticular.

In still another embodiment, compositions of the present invention are formulated as rectal suppositories, which may contain suppository bases including, but not limited to, cocoa butter or glycerides.

In still another embodiment, compositions of the present invention comprise an antiprogestin and a bio-adhesive carrier such as those described in U.S. Patent No. 4,615,697, which is incorporated herein by reference. The bio-adhesive carrier may be in gel, cream, tablet, pill, capsule, suppository, or film form or any other pharmaceutically acceptable form that will adhere to the vaginal mucosa.

Compositions of the present invention may also be formulated for inhalation, which may be in a form including, but not limited to, a solution, suspension, or emulsion that may be administered as a dry powder or in the form of an aerosol using a propellant, such as dichlorofluoromethane or trichlorofluoromethane.

Compositions of the present invention may also be formulated for transdermal delivery, for example as a cream, ointment, lotion, paste, gel, medicated plaster, patch, or membrane. Such compositions can comprise any suitable excipients, for example penetration enhancers and the like.

Compositions of the present invention may also be formulated for parenteral administration including, but not limited to, by injection or continuous infusion. Formulations for injection may be in the form of suspensions, solutions, or emulsions in oily or aqueous vehicles. Such compositions may also be provided in powder form for reconstitution with a suitable vehicle including, but not limited to, sterile, pyrogen-free water, WFI, and the like.

Compositions of the present invention may also be formulated as a depot preparation, which may be administered by implantation or by intramuscular injection. Such compositions may be formulated with suitable polymeric or hydrophobic materials (as an emulsion in an acceptable oil, for example), ion exchange resins, or as sparingly soluble derivatives (as a sparingly soluble salt, for example).

Compositions of the present invention may also be formulated as a liposome preparation. Liposome preparations can comprise liposomes which penetrate the cells of interest or the stratum corneum and fuse with the cell membrane resulting in delivery of the contents of the liposome into the cell. For example, liposomes such as those described in U.S. Patent No. 5,077,211 to Yarosh, U.S. Patent No. 4,621,023 to Redziniak et al.*,* or U.S. Patent No. 4,508,703 to Redziniak et al. can be used.

A composition of the invention can be in the form of solid dosage units such as tablets, (e.g., suspension tablets, bite suspension tablets, rapid dispersion tablets, chewable tablets, effervescent tablets, bilayer tablets.), caplets, capsules (e.g., soft or hard gelatin capsules), powder (e.g., packaged powder, dispensable powder or effervescent powder), lozenges, sachets, cachets, troches, pellets, granules, microgranules, encapsulated microgranules, powder aerosol formulations, or any other solid dosage form reasonably adapted for administration.

Tablets can be prepared according to any of the many relevant, well known pharmacy techniques. In one embodiment, tablets or other solid dosage forms can be prepared by processes that employ one or a combination of methods including, without limitation, (1) dry mixing, (2) direct compression, (3) milling, (4) dry or non-aqueous granulation, (5) wet granulation, or (6) fusion.

The individual steps in the wet granulation process of tablet preparation typically include milling and sieving of the ingredients, dry powder mixing, wet massing, granulation and final grinding. Dry granulation involves compressing a powder mixture into a rough tablet or "slug" on a heavy-duty rotary tablet press. The slugs are then broken up into granular particles by a grinding operation, usually by passage through an oscillation granulator. The individual steps include mixing of the powders, compressing (slugging) and grinding (slug reduction or granulation). Typically, no wet binder or moisture is involved in any of the steps.

In another embodiment, solid dosage forms can be prepared by mixing an antiprogestin with one or more pharmaceutical excipients to form a substantially homogenous pre-formulation blend. The pre-formulation blend can then be subdivided and optionally further processed (e.g., compressed, encapsulated, packaged, dispersed, etc.) into any desired dosage forms.

Compressed tablets can be prepared by compacting a powder or granulation composition of the invention. The term "compressed tablet" generally refers to a plain, uncoated tablet suitable for oral ingestion, prepared by a single compression or by pre-compaction tapping followed by a final compression. Tablets of the present invention may be coated or otherwise compounded to provide a dosage form affording the advantage of improved handling or storage characteristics. In one embodiment, any such coating will be selected so as to not substantially delay onset of therapeutic effect of a composition of the invention upon administration to a subject. The term "suspension tablet" as used herein refers to a compressed tablet that rapidly disintegrates after placement in water.

Suitable liquid dosage forms of a composition of the invention include solutions, aqueous or oily suspensions, elixirs, syrups, emulsions, liquid aerosol formulations, gels, creams, ointments, etc. Such compositions may also be formulated as a dry product for constitution with water or other suitable vehicle before use.

In one embodiment, liquid or semi-solid compositions, upon storage in a closed container maintained at either room temperature, refrigerated (e.g., about 5 -10°C) temperature, or freezing temperature for a period of about 1, 2, 3, 4, 5, 6, 7, 8, 9, 10, 11, or 12 months, exhibit at least about 90%, at least about 92.5%, at least about 95%, or at least about 97.5% of the original antiprogestin compound present therein.

Compositions of the invention can, if desired, include one or more pharmaceutically acceptable excipients. The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a unit dose of the composition. Excipients include, by way of illustration and not limitation, diluents, disintegrants, binding agents, adhesives (e.g., bioadhesives), wetting agents, lubricants, glidants, surface modifying agents or surfactants, fragrances, suspending agents, emulsifying agents, nonaqueous vehicles, preservatives, antioxidants, adhesives, agents to adjust pH and osmolarity (e.g., buffering agents), preservatives, thickening agents, sweetening agents, flavoring agents, taste masking agents, colorants or dyes, penetration enhancers and substances added to improve appearance of the composition.

Excipients optionally employed in compositions of the invention can be solids, semi-solids, liquids or combinations thereof. Compositions of the invention containing excipients can be prepared by any known technique of pharmacy that comprises mixing an excipient with a drug or therapeutic agent.

Compositions of the invention optionally comprise one or more pharmaceutically acceptable diluents as excipients. Suitable diluents illustratively include, either individually or in combination, lactose, including anhydrous lactose and lactose monohydrate; starches, including directly compressible starch and hydrolyzed starches (e.g., Celutab™ and Emdex™); mannitol; sorbitol; xylitol; dextrose (e.g., Cerelose™ 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of α- and amorphous cellulose (e.g., Rexcel™) and powdered cellulose; calcium carbonate; glycine; bentonite; polyvinylpyrrolidone; and the like. Such diluents, if present, constitute in total about 5% to about 99%, about 10% to about 85%, or about 20% to about 80%, of the total weight of the composition. Any diluent or diluents selected preferably exhibit suitable flow properties and, where tablets are desired, compressibility.

The use of extragranular microcrystalline cellulose (that is, microcrystalline cellulose added to a wet granulated composition after a drying step) can be used to improve hardness (for tablets) and/or disintegration time.

Compositions of the invention optionally comprise one or more pharmaceutically acceptable disintegrants as excipients, particularly for tablet, capsule or other solid formulations. Suitable disintegrants include, either individually or in combination, starches, including sodium starch glycolate (e.g., Explotab™ of PenWest) and pregelatinized corn starches (e.g., National™ 1551, National™ 1550, and Colocorn™ 1500), clays (e.g., Veegum™ HV), celluloses such as purified cellulose, microcrystalline cellulose, methylcellulose, carboxymethylcellulose and sodium carboxymethylcellulose, croscarmellose sodium (e.g., Ac-Di-Sol™ of FMC), alginates, crospovidone, and gums such as agar, guar, xanthan, locust bean, karaya, pectin and tragacanth gums.

Disintegrants may be added at any suitable step during the preparation of the composition, particularly prior to a granulation step or during a lubrication step prior to compression. Such disintegrants, if present, constitute in total about 0.2% to about 30%, about 0.2% to about 10%, or about 0.2% to about 5%, of the total weight of the composition.

Compositions of the invention optionally comprise one or more pharmaceutically acceptable binding agents or adhesives as excipients, particularly for tablet formulations. Such binding agents and adhesives preferably impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include, either individually or in combination, acacia; tragacanth; sucrose; gelatin; glucose; starches such as, but not limited to, pregelatinized starches (e.g., National™ 1511 and National™ 1500); celluloses such as, but not limited to, methylcellulose and carmellose sodium (e.g., Tylose™); alginic acid and salts of alginic acid; magnesium aluminum silicate; PEG; guar gum; polysaccharide acids; bentonites; povidone, for example povidone K-15, K-30 and K-29/32; polymethacrylates; HPMC; hydroxypropylcellulose (e.g., Klucel™); and ethylcellulose (e.g., Ethocel™). Such binding agents and/or adhesives, if present, constitute in total about 0.5% to about 25%, about 0.75% to about 15%, or about 1% to about 10%, of the total weight of the composition.

Compositions of the invention optionally comprise one or more pharmaceutically acceptable wetting agents as excipients. Non-limiting examples of surfactants that can be used as wetting agents in compositions of the invention include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (e.g., Labrasol™ of Gattefosse), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (e.g., Tween™80 of ICI), propylene glycol fatty acid esters, for example propylene glycol laurate (e.g., Lauroglycol™ of Gattefosse), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, about 0.4% to about 10%, or about 0.5% to about 5%, of the total weight of the composition.

Compositions of the invention optionally comprise one or more pharmaceutically acceptable lubricants (including anti-adherents and/or glidants) as excipients. Suitable lubricants include, either individually or in combination, glyceryl behapate (e.g., Compritol™ 888); stearic acid and salts thereof, including magnesium (magnesium stearate), calcium and sodium stearates; hydrogenated vegetable oils (e.g., Sterotex™); colloidal silica; talc; waxes; boric acid; sodium benzoate; sodium acetate; sodium fumarate; sodium chloride; DL-leucine; PEG (e.g., Carbowax™ 4000 and Carbowax™ 6000); sodium oleate; sodium lauryl sulfate; and magnesium lauryl sulfate. Such lubricants, if present, constitute in total about 0.1% to about 10%, about 0.2% to about 8%, or about 0.25% to about 5%, of the total weight of the composition.

Suitable anti-adherents include talc, cornstarch, DL-leucine, sodium lauryl sulfate and metallic stearates. Talc is an anti-adherent or glidant used, for example, to reduce formulation sticking to equipment surfaces and also to reduce static in the blend. One or more anti-adherents, if present, constitute about 0.1% to about 10%, about 0.25% to about 5%, or about 0.5% to about 2%, of the total weight of the composition.

Glidants can be used to promote powder flow of a solid formulation. Suitable glidants include colloidal silicon dioxide, starch, talc, tribasic calcium phosphate, powdered cellulose and magnesium trisilicate. Colloidal silicon dioxide is particularly preferred.

Compositions of the present invention can comprise one or more anti-foaming agents. Simethicone is an illustrative anti-foaming agent. Anti-foaming agents, if present, constitute about 0.001 % to about 5%, about 0.001 % to about 2%, or about 0.001 % to about 1%, of the total weight of the composition.

Illustrative antioxidants for use in the present invention include, but are not limited to, butylated hydroxytoluene, butylated hydroxyanisole, potassium metabisulfite, etc. One or more antioxidants, if desired, are typically present in a composition of the invention in an amount of about 0.01% to about 2.5%, for example about 0.01%, about 0.05%, about 0.1%, about 0.5%, about 1%, about 1.5%, about 1.75%, about 2%, about 2.25%, or about 2.5%, by weight.

In various embodiments, compositions of the invention can comprise a preservative. Suitable preservatives include, but are not limited to, benzalkonium chloride, methyl, ethyl, propyl or butylparaben, benzyl alcohol, phenylethyl alcohol, benzethonium, methyl or propyl p-hydroxybenzoate and sorbic acid or combinations thereof. Typically, the optional preservative is present in an amount of about 0.01% to about 0.5% or about 0.01% to about 2.5%, by weight.

In one embodiment, compositions of the invention optionally comprise a buffering agent. Buffering agents include agents that reduce pH changes. Illustrative classes of buffering agents for use in various embodiments of the present invention comprise a salt of a Group IA metal including, for example, a bicarbonate salt of a Group IA metal, a carbonate salt of a Group IA metal, an alkaline or alkali earth metal buffering agent, an aluminum buffering agent, a calcium buffering agent, a sodium buffering agent, or a magnesium buffering agent. Suitable buffering agents include carbonates, phosphates, bicarbonates, citrates, borates, acetates, phthalates, tartrates, succinates of any of the foregoing, for example sodium or potassium phosphate, citrate, borate, acetate, bicarbonate and carbonate.

Non-limiting examples of suitable buffering agents include aluminum, magnesium hydroxide, aluminum glycinate, calcium acetate, calcium bicarbonate, calcium borate, calcium carbonate, calcium citrate, calcium gluconate, calcium glycerophosphate, calcium hydroxide, calcium lactate, calcium phthalate, calcium phosphate, calcium succinate, calcium tartrate, dibasic sodium phosphate, dipotassium hydrogen phosphate, dipotassium phosphate, disodium hydrogen phosphate, disodium succinate, dry aluminum hydroxide gel, magnesium acetate, magnesium aluminate, magnesium borate, magnesium bicarbonate, magnesium carbonate, magnesium citrate, magnesium gluconate, magnesium hydroxide, magnesium lactate, magnesium metasilicate aluminate, magnesium oxide, magnesium phthalate, magnesium phosphate, magnesium silicate, magnesium succinate, magnesium tartrate, potassium acetate, potassium carbonate, potassium bicarbonate, potassium borate, potassium citrate, potassium metaphosphate, potassium phthalate, potassium phosphate, potassium polyphosphate, potassium pyrophosphate, potassium succinate, potassium tartrate, sodium acetate, sodium bicarbonate, sodium borate, sodium carbonate, sodium citrate, sodium gluconate, sodium hydrogen phosphate, sodium hydroxide, sodium lactate, sodium phthalate, sodium phosphate, sodium polyphosphate, sodium pyrophosphate, sodium sesquicarbonate, sodium succinate, sodium tartrate, sodium tripolyphosphate, synthetic hydrotalcite, tetrapotassium pyrophosphate, tetrasodium pyrophosphate, tripotassium phosphate, trisodium phosphate, and trometarnol. (based in part upon the list provided in The Merck Index, Merck & Co. Rahway, N.J. (2001)). Furthermore, combinations or mixtures of any two or more of the above-mentioned buffering agents can be used in the pharmaceutical compositions described herein. One or more buffering agents, if desired, are present in compositions of the invention in an amount of about 0.01% to about 5% or about 0.01% to about 3%, by weight.

In various embodiments, compositions the invention may include one or more agents that increase viscosity. Illustrative such agents include, but are not limited to, methylcellulose, carboxy-methylcellulose sodium, ethylcellulose, carrageenan, carbopol, and/or combinations thereof. Typically, one or more viscosity increasing agents are present in compositions of the invention in an amount of about 0.1% to about 10%, or about 0.1% to about 5%, by weight.

In various embodiments, compositions of the invention comprise an "organoleptic agent" to improve the organoleptic properties of the composition. The term "organoleptic agent" herein refers to any excipient that can improve the flavor or odor of, or help mask a disagreeable flavor or odor of a composition of the invention. Such agents include sweeteners, flavoring agents and/or taste masking agents. Suitable sweeteners and/or flavoring agents include any agent that sweetens or provides flavor to a pharmaceutical composition. Optional organoleptic agents are typically present in a composition of the invention in an amount of about 0.1 mg/ml to about 10 mg/ml, about 0.5 mg/ml to 5 mg/ml or about I mg/ml.

Illustrative sweeteners or flavoring agents include, without limitation, acacia syrup, anethole, anise oil, aromatic elixir, benzaldehyde, benzaldehyde elixir, cyclodextrins, caraway, caraway oil, cardamom oil, cardamom seed, cardamom spirit, cardamom tincture, cherry juice, cherry syrup, cinnamon, cinnamon oil, cinnamon water, citric acid, citric acid syrup, clove oil, cocoa, cocoa syrup, coriander oil, dextrose, eriodictyon, eriodictyon fluidextract, eriodictyon syrup, aromatic, ethylacetate, ethyl vanillin, fennel oil, ginger, ginger fluidextract, ginger oleoresin, dextrose, glucose, sugar, maltodextrin, glycerin, glycyrrhiza, glycyrrhiza elixir, glycyrrhiza extract, glycyrrhiza extract pure, glycyrrhiza fluid extract, glycyrrhiza syrup, honey, iso-alcoholic elixir, lavender oil, lemon oil, lemon tincture, mannitol, methyl salicylate, nutmeg oil, orange bitter, elixir, orange bitter, oil, orange flower oil, orange flower water, orange oil, orange peel, bitter, orange peel sweet, tincture, orange spirit, orange syrup, peppermint, peppermint oil, peppermint spirit, peppermint water, phenylethyl alcohol, raspberry juice, raspberry syrup, rosemary oil, rose oil, rose water, stronger, saccharin, saccharin calcium, saccharin sodium, sarsaparilla syrup, sarsaparilla, sorbitol solution, spearmint, spearmint oil, sucrose, sucralose, syrup, thyme oil, tolu balsam, tolu balsam syrup, vanilla, vanilla tincture, vanillin, wild cherry syrup, or combinations thereof.

Illustrative taste masking agents include, but are not limited to, cyclodextrins, cyclodextrin emulsions, cyclodextrin particles, cyclodextrin complexes, or combinations thereof.

Illustrative suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats.

Illustrative emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol.

The foregoing excipients can have multiple roles as is known in the art. For example, starch can serve as a filler as well as a disintegrant. The classification of excipients above is not to be construed as limiting in any manner.

Compositions of the present invention may be administered in any manner including, but not limited to, orally, parenterally, sublingually, transdermally, rectally, transmucosally, topically, via inhalation, via buccal administration, or combinations thereof. Parenteral administration includes, but is not limited to, intravenous, intraarterial, intraperitoneal, subcutaneous, intramuscular, intrathecal, intraarticular, intracisternal and intraventricular.

A therapeutically effective amount of the composition required for use in therapy varies with the length of time that activity is desired, and the age and the condition of the patient to be treated, among other factors, and is ultimately determined by the attendant physician. In general, however, doses employed for human treatment typically are in the range of about 0.001 mg/kg to about 500 mg/kg per day, for example about 1 µg/kg to about 1 mg/kg per day or about 1 µg/kg to about 100 µg/kg per day. For most large mammals, the total daily dosage is from about 1 to 100 mg, preferably from about 2 to 80 mg. The dosage regimen may be adjusted to provide the optimal therapeutic response. The desired dose may be conveniently administered in a single dose, or as multiple doses administered at appropriate intervals, for example as two, three, four or more sub-doses per day.

Illustratively, a composition of the invention may be administered to a subject to provide the subject with an antiprogestin in an amount of about 1 µg/kg to about 1 mg/kg body weight, for example about 1 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg or about 1 mg/kg body weight.

Patients undergoing treatments with the compositions of the instant invention should be monitored routinely for their serum estrogen and glucocorticoid levels.

The following non-limiting examples are provided to aid in understanding the teachings of the instant invention.

### Example 1. Formulations of the instant invention can be prepared as tablets

To obtain tablets for practicing the instant invention, the following ingredients can be pressed together in a tablet press:
10.0 mg 21-methoxy-11β-(4-N,N-dimethylaminophenyl)-19-norpregna-4,9-diene-3,20-dione
140.5 mg lactose
69.5 mg corn starch
2.5 mg poly-N-vinylpyrrolidone
2.0 mg aerosol
0.5 mg magnesium stearate

To obtain oily preparations for practicing the instant invention, for example the following ingredients can be mixed together and loaded into ampoules:
100.0 mg 21-methoxy-11β-(4-N,N-dimethylaminophenyl)-19-norpregna-4,9-diene-3,20-dione
343.4 mg castor oil
608.6 mg benzyl benzoate

### Example 2. Measuring in vitro binding affinities of antiprogestins

Competitive binding assays are performed using cytosolic preparations.

For measuring binding to rabbit progesterone receptor (PR) and glucocorticoid receptor (GR), cytosol is prepared from uterus or thymus, respectively, of estradiol-primed immature rabbits. For binding to rabbit uterine PR, cytosol containing rabbit uterine PR is prepared in TEGMD buffer (10 mM Tris, pH 7.2, 1.5 mM EDTA, 0.2 mM sodium molybdate, 10% glycerol, 1 mM DTT) and incubated with 6 nM 1,2-[³H] progesterone (NEN Life Science Products; 52 Ci/mmol); test compounds are added at concentrations from 2 to 100 nM. For binding to rabbit thymic GR, cytosol is prepared in TEGMD buffer and incubated with 6 nM 6,7-[³H] dex (NEN; 35 or 40 Ci/mmol); test compounds are added at concentrations from 2 to 100 nM.

For measuring binding to human progesterone receptor-A (rhPR-A) or progesterone receptor-B (rhPR-B), cytosolic extracts from Sf9 insect cells infected with recombinant baculo-virus expressing either hPR-A or hPR-B is prepared. Sf9 cytosol (prepared in TEGMD buffer containing the following protease inhibitors: bacitracin at 100 µg/ml, aprotinin at 2 µg/ml, leu-peptin at 94 µg/ml, pepstatin A at 200 µg/ml) is incubated with 6.8 nM 1,2,6,7,16,17-[³H] progesterone (NEN; 143 Ci/mmol); test compounds are added at concentrations from 1 to 100 nM.

After overnight incubation at 4 C, bound and unbound [³H]-steroids are separated by addition of dextran-coated charcoal and centrifugation at 2100 x g for 15 minutes at 4°C. Supernatants from GR assays are decanted and counted in a Beckman LS-1800 liquid scintillation counter. Supernatants containing PR are pipetted into 24-well microplates and counted in a Packard TopCount liquid scintillation counter. Counts per minute (cpm) are entered into Packard's RIASmart™ for calculation of EC₅₀'s. Relative binding affinity for each test compound is calculated as follows: (EC₅₀ of standard)/(EC₅₀ of competitor) x 100. The standard for the PR binding assays is P4 and the standard for the GR binding assays is dex.

### Example 3. Measuring antiglucocorticoid and progesterone antagonist activity in vivo

For measuring *in vivo* progesterone antagonist activity of test compounds, T47D-CO human breast cancer cells, grown in monolayer culture in phenol red-free DMEM supplemented with 10% fetal bovine serum (FBS), 10 U/ml penicillin G and 10 µg/ml streptomycin sulfate, are transfected with a suitable hormone sensitive reporter gene plasmid, for example PRE₂-tk-LUC, which contains two copies of a progestin/ glucocorticoid/androgen response element upstream of the thymidine kinase (tk) promoter and the firefly luciferase (LUC) reporter gene. Transfected T47D-CO cells are incubated with a (predetermined) maximum stimulatory concentration of a progestogen, for example P₄, in the absence or presence of various concentrations of test compound for 20 hours. LUC activity is determined using Promega's Luciferase Assay System and the IC₅₀ of the test compound is determined.

For measuring *in vivo* glucocorticoid antagonist activity, HepG2 human hepatoblastoma cells, grown in monolayer culture in phenol red-free MEMα supplemented with 10% FBS and pen/strep, are co-transfected with a suitable hormone sensitive reporter gene plasmid such as PRE₂-tk-LUC and a GR expression plasmid. Transfected HepG2 cells are incubated with a (predetermined) maximum stimulatory concentration of dexamethasone in the absence or presence of various concentrations of test compound for 20 hours. IC₅₀ of the test compound is determined by measuring LUC activity.

### Example 4. Chronic daily administration of CDB-4124 is associated with toxic liver effects

Initial studies conducted with Proellex (aka CDB-4124) demonstrated efficacy of the drug at every dose tested. Development of Proellex has focused on the two highest doses tested, 25 mg and 50 mg based on data suggesting that higher doses suppressed endometrial thickening and the potential for breakthrough uterine bleeding. Neither animal preclinical studies nor small trials in women in Europe at the higher doses for periods of up to six months of exposure predicted the liver toxicity exhibited in the Phase III clinical studies conducted in a diverse population in the United States. Proellex, delivered orally at a dose of 50 mg/day, exhibited severe liver toxicity in roughly 3-4% of the women receiving this dose. At 12.5 mg there were no adverse liver toxicity signals different from placebo. The maximum concentrations of CDB-4124 and its mono-demethylated metabolite (CDB-4453) for the 12.5 mg dose were 25% of the 50 mg dose. All liver toxicities resolved in those women that returned for safety follow-ups, including those subjects that developed liver-associated serious adverse effects (SAEs). The effects observed when Proellex was administered orally at 50 mg/day were significantly lower in frequency and intensity when Proellex was delivered at 25 mg/day. This observation was further amplified by the fact that longer durations of exposure have been safely achieved at a 25 mg/day dose than at a 50 mg/day dose suggesting that duration of exposure at lower doses does not necessarily result in the same liver toxicity than that observed at the 50 mg/day dose.

To date, over 600 patients, including women with confirmed cases of endometriosis or uterine fibroids, have participated in double blind and open label clinical trials in which patients were administered daily oral capsules containing doses of 12.5 mg, 25 mg or 50 mg CDB-4124 (Proellex) for over one month. Of these patients, about 500 received Proellex and about 130 received a placebo. Of the patients receiving Proellex, about 190 received a dose of 50 mg CDB-4124 per day, about 260 received a dose of 25 mg CDB-4124 per day and about 55 received a dose of 12.5 mg per day.

Liver enzymes were frequently monitored in participating subjects. The liver enzyme level at which the clinical trials would be discontinued was set at an increase in liver aminotransferases greater than, or equal to three times the Upper Limit of Normal (≥ 3 x ULN).

During clinical trials, thirteen subjects were found to exhibit an increase in liver enzymes ≥ 3 x ULN, but this was confirmed by a repeat test in 48 hours in only nine subjects. Of the nine subjects with a confirmed increase in liver enzymes ≥ 3 x ULN, seven were severe enough elevations to be reported to the FDA as SAEs. One of these seven subjects had been receiving a dose of 25mg CDB-4124 per day; the remaining six subjects had been receiving a dose of 50mg CDB-4124 per day. Liver enzymes ≥ 3 x ULN persisted in five of the nine subjects with a confirmed increase in liver enzymes ≥ 3 x ULN. These five subjects had previously been dosed with the 50mg dose. One of these subjects is receiving oral medication for treatment of her liver condition. Clinical trials involving CDB-4124 at all doses were voluntarily suspended as a result of these SAEs and were subsequently placed on clinical hold by the United States Food and Drug Administration for safety reasons.

Pharmacokinetic studies performed on participating subjects detected a high Cₘₐₓ and a Tₘₐₓ at 1-2 hours following administration. Large quantities of the monodemethylated metabolite of CDB-4124 were also detected, clearly indicating first pass metabolism of the antiprogestin. Providing further evidence of first pass metabolism, primary cultures of human and animal hepatocytes rapidly produce the mono-demethylated metabolite of CDB-4124. Metabolism of CDB-4124 by the liver provides the opportunity for liver damage and greatly reduces the concentration of the antiprogestin before it reaches the systemic circulation. Thus, alternative routes of administration of antiprogestins that avoid first pass metabolism such as, without limitation, intravenous, intramuscular, and sublingual, should allow antiprogestins to be absorbed directly into the systemic circulation and thereby provide a method for treating progestone-dependent conditions while avoiding liver toxicity. Administration routes which avoid first pass metabolism may also require less drug per dose to achieve the same therapeutic benefit relative to oral administration.

Pre-clinical studies were performed on rodents with breast tumors induced by 7,12-Dimethylbenz(a)anthracene (DMBA). These studies demonstrated efficacy of non-oral delivery of CDB-4124. In particular, CDB-4124 delivered by subcutaneous injection was effective in reducing the quantity and size of DMBA-induced breast tumors providing proof of concept.

### Example 5. Vaginal dDelivery of CDB-4124 and CDB-4453 reduces systemic concentrations compared to oral administration and avoids first pass metabolism

Beagles were administered 25 mg of CDB-4124 or CDB-4453 (the mono-demethylated metabolite of CDB-4124) formulated as either a micronized powder or a vaginal suppository. As illustrated at Figure 1, CDB-4124 and CDB-4453, when administered orally as a micronized powder, are rapidly metabolized after a peak plasma concentration (Cmax) is achieved. In contrast, when the same compounds are administered locally via vaginal suppository, the drugs are metabolized slowly and peak plasma concentrations (Cmax) are relatively low. Moreover, systemic exposure of the drug is much lower when administered locally (compare AUC for CDB-4124 and CDB-4453 when administered vaginally vs. orally).

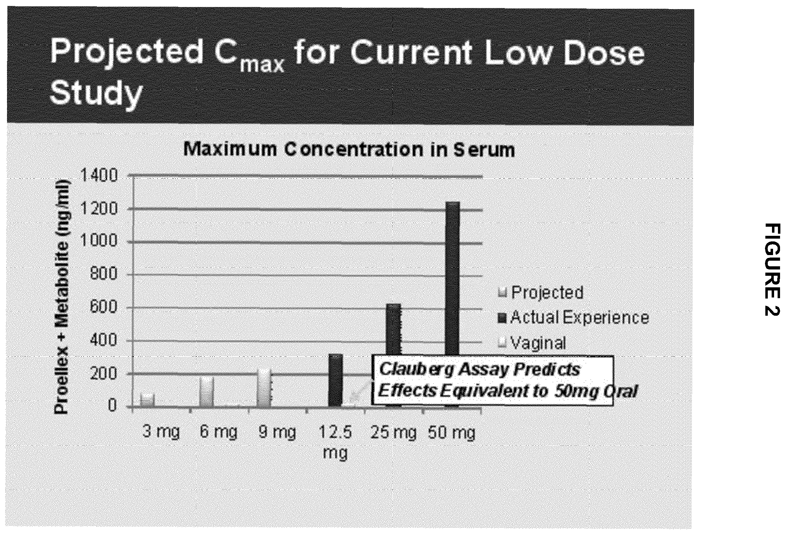

The maximum circulating concentrations (Cmax) of CDB-4124 obtained following vaginal administration to beagles were extrapolated to humans for the 12.5mg, 25mg and 50 mg doses actually administered during the Phase III clinical studies. As can be seen from Fig. 2, the predicted Cmax for vaginal administration of the 12.5 mg dose of CDB-4124 in humans is approximately 6.5% of the same dose when administered orally and the predicted Cmax for vaginal administration of the 50 mg dose of CDB-4124 in humans is approximately 2% of the same dose when administered orally.

### Example 6. Bioavailability of CDB-4124 at the uterus is surprisingly low when administered orally

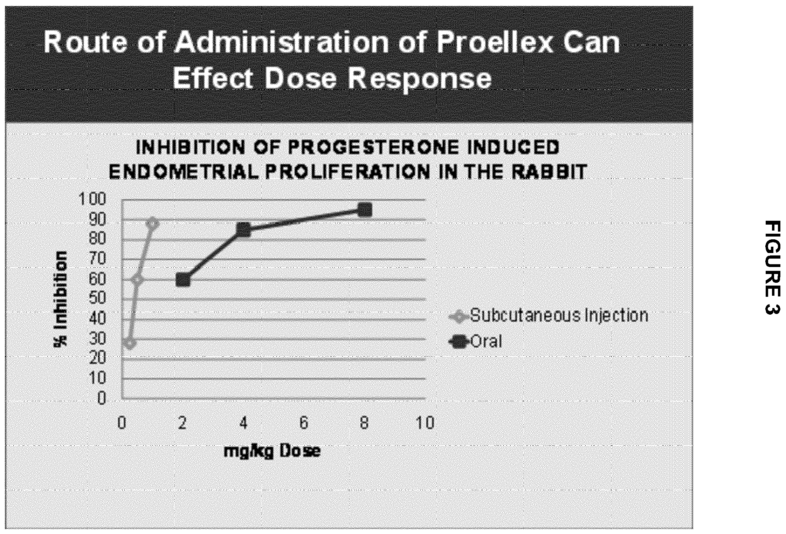

To determine whether the low circulating levels of CDB-4124 when administered locally could have any impact predictive of efficacy, an anti-Clauberg study was run in which immature estradiol-primed rabbits were co-administered progesterone and various doses of CDB-4124 by either subcutaneous or oral administration. At least 3 different highly trained individuals evaluated the rabbit uterus for glandular growth, for complexity and overall progesterone-induced "development". The inhibition (by percentage) of progesterone-induced endometrial proliferation at each dose was assayed. As illustrated at Figure 3, maximal inhibition was observed at a dose of less than 1 mg/kg when CDB-4124 was administered subcutaneously. However, maximal inhibition required a -8-fold increase in dosage when administered orally (i.e., 8 mg/kg). Importantly 8 mg/kg corresponds closely to the 50mg/day dose of CDB-4124 administered to the female subjects described in Example 4. This demonstrates that the effective local concentration of CDB-4124 at the endometrium is greatly decreased when the drug is administered orally, most likely due to first-pass metabolism of the drug. Accordingly, in order to achieve therapeutic effect, e.g., for indications localized to the pelvic and reproductive tract, a relatively high dosage of CDB-4124 is required when administered orally, corresponding closely to the dosage of CDB-4124 at which toxic liver effects were observed in Example 4.

Another anti-Clauberg study was run in which immature estradiol-primed rabbits were administered progesterone alone (vehicle control) or were co-administered progesterone and three doses of CDB-4124 by either vaginal or oral administration. The inhibition of progesterone-induced endometrial proliferation at each dose was assayed. Fig. 3 illustrates the decrease in the McPhail index following increasing doses of CDB-4124 administered by either route. Maximal inhibition (i.e., a decrease in the McPhail index to 1.5) occurred at 0.2 mg/kg CDB-4124 when administered vaginally, compared to 0.8 mg/kg when administered orally. The data from this study show that vaginal delivery of CDB-4124 exhibits four times the antiprogestational activity of the same oral dose.

Cumulatively, the data indicate that a four-fold lower dose of antiprogestin can be administered vaginally compared to the effective dose when orally administered, while attaining only a small fraction of the maximal circulating concentrations compared to oral administration, thereby avoiding liver toxicity. For example, equivalent antiprogestational activity at the uterus is observed for a 50 mg oral dose of CDB-4124 and a 12.5 mg vaginal dose; however, the Cmax observed with a 12.5 mg vaginal dose is only 2% that observed with a 50mg oral dose. The relatively high local concentration of the drug achieved by local administration allows a relatively low dose of the drug (compared with oral administration) to achieve therapeutic effect for indications localized to the pelvic and reproductive tract (e.g. endometriosis, uterine fibroids and ovarian cancer). Because a high concentration of the drug in the systemic circulation (and associated first pass metabolism of the drug) is not reached by local administration, avoidance of the severe liver toxicity observed in a small percentage of subjects following oral administration of CDB-4124 in previous Phase III clinical studies at doses of 25 and 50 mg is a surprising advantage of administering the drug locally. Similar advantages should inure to local administration of other antiprogestins.

The present invention is further illustrated by the following embodiments (abbreviated as "EMB" in the following):
EMB 1: A compound having the general formula: 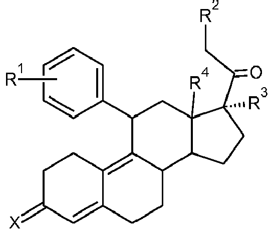 or a pharmaceutically acceptable salt thereof wherein: R¹ is selected from the group consisting of: alkyl; alkenyl; cycloalkyl; cycloalkenyl; aryl; H; CH₃SO; CH₃SO₂; acyl; alkoxy; thioalkoxy; thioalkyl, acyloxy; Si(CH₃)₃; 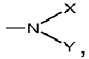 wherein X and Y are acyl; aziridinyl, azirinyl, azetidinyl, pyrrolidinyl, ethoxypyrrolidinyl, methoxypyrrolidinyl, piperidinyl, ethoxypiperidinyl, morpholinyl, ethoxymorpholinyl, oxazinyl, piperazinyl, methylpiperazinyl, ethylpiperazinyl, and diazinyl; R² is selected from the group consisting of: hydrogen, halogen, alkyl, acyl, hydroxyl, akoxy, acyloxy, alkyl carbonate, cypionyloxy, S-alkyl, S-CN, S-acyl and -OC(O)R⁶ wherein R⁶ is alkyl, alkoxyalkyl or alkoxy; R³ is selected from the group consisting of: alkyl, hydroxy, alkoxy, and acyloxy with the proviso that R³ is other than acetoxy or propynyl; R⁴ is hydrogen or alkyl; and X is selected from the group consisting of: =O, =N-OR5 wherein R5 is hydrogen or alkyl, OH, CH₂, OAlk₁, and OCOAlk₂, wherein Alk₁ and Alk₂ are C1-C8 alkyl or C7-C15 aralalkyl, with the proviso that if R¹ is at the para position and is -OCH₃, -SCH₃, -NC₄H₈, - NC₅H₁₀, -NC₄H₈O, -CHO, -CH(OH)CH₃, -COCH₃, -O(CH₂)₂NC₄H₈, or -O(CH₂)₂NC₅H₁₀, X is other than =O or =N-OR5 wherein R5 is hydrogen or alkyl.
EMB 2: A compound or salt thereof in accordance with EMB 1, wherein R₁ is at the para position and is selected from the group consisting of -OCH₃, -SCH₃, -NC₄H₈, -NC₅H₁₀, - NC₄H₈O, -CHO, -CH(OH)CH₃, -COCH₃, -O(CH₂)₂NC₄H₈ and -O(CH₂)₂NC₅H₁₀; and X is selected from the group consisting of OH, CH₂, OAlk1, and OCOAlk2, wherein Alk1 and Alk2 are C1-C8 alkyl or C7-C15 aralalkyl.
EMB 3: A compound or salt thereof in accordance with EMB 1, wherein R¹ is at the ortho or meta position and is selected from the group consisting of -OCH₃, -SCH₃, -NC₄H₈, - NC₅H₁₀, -NC₄H₈O, -CHO, -CH(OH)CH₃, -COCH₃, -O(CH₂)₂NC₄H₈ and -O(CH₂)₂NC₅H₁₀.
EMB 4: A compound or salt thereof in accordance with EMB 1, wherein R¹ is at the para position and is selected from aziridinyl, azirinyl, azetidinyl, methoxypyrrolidinyl, ethoxymorpholinyl, oxazinyl, piperazinyl, methylpiperazinyl, ethylpiperazinyl and diazinyl.
EMB 5: A compound or salt thereof in accordance with EMB 1, wherein R¹ is at the ortho or meta position and is selected from aziridinyl, azirinyl, azetidinyl, methoxypyrrolidinyl, ethoxymorpholinyl, oxazinyl, piperazinyl, methylpiperazinyl, ethylpiperazinyl and diazinyl.
EMB 6: A pharmaceutical composition comprising a therapeutically effective amount of a compound or salt thereof according to EMB 1 and a pharmaceutically acceptable excipient.
EMB 7: A method for producing an antiprogestational effect in a patient, comprising administering to said patient a therapeutically effective amount of a compound or salt thereof in accordance with EMB 1.
EMB 8: A method for treating a progesterone-dependent condition selected from the group consisting of endometriosis and pain associated therewith, adenomyosis, endometriomas of the ovary, dysmenorrhea, uterine fibroids, endometrial hyperproliferation, ovarian cancer, and cervical cancer comprising administering to a patient in need thereof a therapeutically effective amount of a compound or salt thereof in accordance with EMB 1.
EMB 9: A method for treating a progesterone-dependent condition selected from the group consisting of endometriosis and pain associated therewith, adenomyosis, endometriomas of the ovary, dysmenorrhea, uterine fibroids, endometrial hyperproliferation, ovarian cancer, and cervical cancer comprising administering to a patient in need thereof a composition in accordance with EMB 6.
EMB 10: The method of EMB 9, wherein the composition is administered via a route selected from the group consisting of: vaginal, intrauterine and topical and wherein the effective amount is less than the effective amount when administered systemically.
EMB 11: The method of EMB 10, wherein the composition is in a form suitable for vaginal administration.
EMB 12: The method of EMB 11, wherein the composition is in the form of a vaginal suppository, a gel or a cream.
EMB 13: The method of EMB 11, wherein the composition is administered locally to the vaginal mucosa of the patient.
EMB 14: A method for treating a progesterone-dependent condition selected from the group consisting of endometriosis and pain associated therewith, adenomyosis, endometriosis or pain associated therewith, dysmenorrhea, uterine fibroids, endometrial hyperproliferation, ovarian cancer, and cervical cancer comprising administering a therapeutically effective amount of a composition comprising an antiprogestin locally to the vaginal mucosa of a patient in need thereof for period of at least 4 months.
EMB 15: The method of EMB 14, wherein the progesterone-dependent condition is endometriosis or uterine fibroids.
EMB 16: The method of EMB 14, wherein the effective amount of the antiprogestin is less than the effective amount when administered systemically.
EMB 17: The method of EMB 14, wherein the composition is in the form of a vaginal suppository, a dissolvable vaginal insert, an intra-uterine device, a topical gel, a transdermal patch and an ointment.
EMB 18: The method of EMB 14 wherein the composition is administered daily.
EMB 19: The method of EMB 14, wherein the composition is administered intermittently.
EMB 20: The method of EMB 14, wherein the antiprogestin is a compound of general formula: 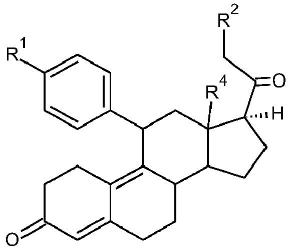 or a pharmaceutically acceptable salt, hydrate or solvate thereof, wherein:
   R¹ is selected from the group consisting of -N(CH₃)₂ and -NHCH₃;
   R² is selected from the group consisting of halogen, alkyl, acyl, hydroxy, alkoxy, acyloxy, alkyl carbonate, cypionyloxy, S-alkyl and S-acyl; and
   R⁴ is selected from the group consisting of hydrogen and alkyl.
EMB 21: The method of EMB 20, wherein said antiprogestin is administered at a dosage from 0.5mg/kg to 500mg/kg.
EMB 22: The method of any of EMB 21, wherein said composition is administered daily at a dosage of 50 mg.
EMB 23: The method of EMB 20, wherein the compound is 21-methoxy-17α-acetoxy-11β-(4 N, N-dimethylaminophenyl)-19-norpregna-4,9-diene-3,20-dione.

## Claims

1. A pharmaceutical composition comprising 17α-acetoxy-11β-(4-N,N-dimethylaminophenyl)-19-norpregna-4,9-diene-3,20-dione for use in treating endometriosis or pain associated therewith, or uterine fibroids, by daily administration of 5 mg to 20 mg of 17α-acetoxy-11β-(4-N,N-dimethylaminophenyl)-19-norpregna-4,9-diene-3,20-dione to the vaginal mucosa of a female patient in need thereof for a period of at least 4 months.

2. The pharmaceutical composition for the use according to claim 1, wherein the amount of 17α-acetoxy-11β-(4-N,N-dimethylamino-phenyl)-19-norpregna-4,9-diene-3,20-dione ad-ministered is 2-fold to 10-fold less than the effective amount when administered systemically.

3. The pharmaceutical composition for the use according to claim 1 or 2, wherein the composition comprises a bio-adhesive carrier and is in the form of a gel, a cream, a tablet, a pill, a capsule or a suppository.

4. The pharmaceutical composition for the use according to claim 1 or 2, wherein the composition is in the form of a solid dosage unit selected from a tablet, a caplet, and a capsule.

5. The pharmaceutical composition for the use according to claim 1 or 2, wherein the composition is for use in treating endometriosis or pain associated therewith.

6. The pharmaceutical composition for the use according to claim 1 or 2, wherein the composition is for use in treating uterine fibroids.

7. The pharmaceutical composition for the use according to any one of claims 1 to 6, wherein the composition is to be administered for a period of at least 12 months.

8. The pharmaceutical composition for the use according to any one of claims 1 to 7, wherein the composition is to be administered intermittently.
